(19) **Europäisches Patentamt / European Patent Office / Office européen des brevets**

(11) **EP 2 008 988 A1**

(12) # EUROPEAN PATENT APPLICATION
published in accordance with Art. 153(4) EPC

(43) Date of publication:
**31.12.2008 Bulletin 2009/01**

(21) Application number: **07739985.5**

(22) Date of filing: **28.03.2007**

(51) Int Cl.:
**C07C 4/18** (2006.01)   **B01J 29/48** (2006.01)
**C07C 5/27** (2006.01)   **C07C 15/04** (2006.01)
**C07C 15/06** (2006.01)   **C07C 15/08** (2006.01)
**C07B 61/00** (2006.01)

(86) International application number:
**PCT/JP2007/056547**

(87) International publication number:
**WO 2007/114127 (11.10.2007 Gazette 2007/41)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **29.03.2006 JP 2006091948**

(71) Applicant: **TORAY INDUSTRIES, INC.**
**Tokyo 103-8666 (JP)**

(72) Inventors:
• **ICHIOKA, Ryoji**
  **Nagoya-shi, Aichi 458-0044 (JP)**

• **MINOMIYA, Eiichi**
  **Okazaki-shi, Aichi 444-3505 (JP)**
• **YAMAKAWA, Shinobu**
  **Nagoya-shi, Aichi 457-0855 (JP)**

(74) Representative: **Webster, Jeremy Mark et al**
   **Mewburn Ellis LLP**
   **York House**
   **23 Kingsway**
   **London**
   **WC2B 6HP (GB)**

(54) **METHOD FOR CONVERSION OF ETHYLBENZENE AND PROCESS FOR PRODUCTION OF PARA-XYLENE**

(57)   A process for converting ethylbenzene, by which ethylbenzene in a feedstocks containing C8 aromatic hydrocarbon is converted to benzene at a high degree of conversion is disclosed. The process for converting ethylbenzene comprises the step of bringing a C8 aromatic hydrocarbon mixed feedstocks containing ethylbenzene into contact with an acid type catalyst containing at least one metal selected from the group consisting of the metals belonging to Group VII and Group VIII in the presence of $H_2$ to convert ethylbenzene to benzene. The feedstocks contains C9-C10 aromatic hydrocarbons including ethyltoluene, and the ethyltoluene is converted to toluene together with the conversion of ethylbenzene.

Fig. 1

**Description**

Technical Field

The present invention relates to a process for converting ethylbenzene and process for producing *p*-xylene. More particularly, the present invention relates to a process for converting ethylbenzene contained in C8 aromatic hydrocarbons by hydrogenation and deethylation, and to a process for producing *p*-xylene by converting ethylbenzene contained in C8 aromatic hydrocarbons by hydrogenation and deethylation and by separating *p*-xylene after isomerization of xylene.

Background Art

Among xylene isomers, the most important one is *p*-xylene. At present, *p*-xylene is used as a material for producing terephthalic acid, which is a monomer constituting polyesters that are ranked with nylons as major polymers, and its demand is high especially in Asia in recent years.

Usually, *p*-xylene is produced from the C8 aromatic hydrocarbon mixture obtained by reforming of naphtha followed by extraction of aromatic hydrocarbons or distillation, or obtained by subjecting the cracked gasoline produced in thermal decomposition of naphtha as a byproduct to extraction of aromatic hydrocarbons or to distillation. Although the composition of the C8 aromatic hydrocarbon mixture material largely varies, it usually contains 10 to 40% by weight of ethylbenzene, 12 to 25% by weight of *p*-xylene, 30 to 50% by weight of *m*-xylene and 12 to 25% by weight of *o*-xylene. Since the C8 aromatic hydrocarbon mixture material usually contains high boiling components having not less than 9 carbon atoms, the high boiling components are removed by distillation, and the resulting C8 aromatic hydrocarbons are supplied to p-xylene-separating step to separate and recover p-xylene. However, since the boiling points of *p*-xylene and *m*-xylene are 138.4°C and 139°C, respectively, and so the difference therebetween is only about 1°C, recovery of *p*-xylene by distillation separation is very ineffective in industrial production. Therefore, *p*-xylene is usually separated by crystallizing separation utilizing the difference between the melting points, or by adsorbing separation utilizing the difference in degree of adsorption to zeolite adsorbent. The C8 aromatic hydrocarbons after the separation step, which is poor in *p*-xylene, is transferred to an isomerization step, where the xylenes are isomerized to a *p*-xylene concentration close to that in the thermodynamic equilibrium composition mainly by a zeolite catalyst. After removing the low boiling byproducts by distillation separation, the resulting hydrocarbon mixture is mixed with the above-described fresh C8 aromatic hydrocarbon material, and the resultant mixture is recycled to the distillation tower where high boiling products having not less than 9 carbon atoms are removed by distillation, followed by separating and recovering *p*-xylene again in the p-xylene-separation step. This series of cycle is hereinafter referred to as "separation-isomerization cycle".

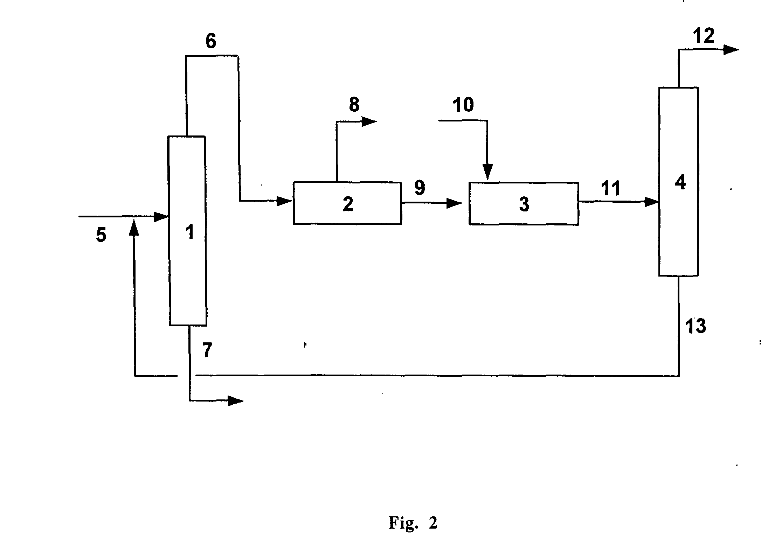

Fig. 2 is a flow chart showing this "separation-isomerization cycle". This "separation-isomerization cycle" comprises a high boiling components-distillation separation step 1 in which the C8 aromatic hydrocarbons contained in the C8 aromatic hydrocarbon mixed feedstocks (hereinafter referred to as "fresh feedstocks") obtained from a reformer or the like and in the recycled material from the isomerization step are recovered and the high boiling components are removed therefrom; a *p*-xylene-separation step 2 in whichp-xylene as a final product is separated; a xylene-isomerization step 3 in which the isomerization of xylenes and conversion of ethylbenzene in the C8 aromatic hydrocarbon material (hereinafter referred to as "raffinate xylene") having a low *p*-xylene concentration is carried out; and a low boiling products-distillation separation step 4 in which low boiling components such as benzene and toluene, produced as byproducts in the isomerization step are separated and recovered. First, the C8 aromatic hydrocarbon mixed feedstocks is transferred to the high boiling components-distillation separation step 1 from the supply line denoted by stream 5, and the high boiling components are removed through the line denoted by stream 7. The C8 aromatic hydrocarbon feed from which the high boiling components have been removed is transferred to the p-xylene-separation step 2 through the line denoted by stream 6, and the *p*-xylene as a final product is separated and recovered from the line denoted by stream 8. The C8 aromatic hydrocarbon feed having a low *p*-xylene concentration is transferred to the xylene-isomerization step 3 through the line denoted by stream 9, in which ethylbenzene is converted to benzene or to xylene through C8 naphthene paraffin as hereinbelow described, and the raffinate xylene having a lowp-xylene concentration is isomerized to attain ap-xylene concentration close to that in the thermodynamic equilibrium composition. To the isomerization step, hydrogen or a hydrogen-containing gas is also supplied through the line denoted by stream 10. The C8 aromatic hydrocarbons from the isomerization step, containing byproducts is transferred to the low boiling products-distillation separation step 4 through the line denoted by stream 11, in which the low boiling components such as benzene and toluene produced as byproducts in the isomerization step are separated and removed through the line denoted by stream 12. The p-xylene-rich recycle feed containing high boiling components is transferred to the high boiling components-distillation separation step 1 through the line denoted by stream 13. In this high boiling components-distillation separation step 1, high boiling components are removed, and the resulting product is recycled again to the p-xylene-separation step 2. There is an option in which one distillation tower is incorporated in the "separation-isomerization cycle" to simultaneously produce o-xylene.

As described above, the C8 aromatic hydrocarbons feedstocks supplied to the separation-isomerization cycle" contain a considerable amount of ethylbenzene. However, in the above-described "separation-isomerization cycle", the ethylbenzene is not removed and remains in the cycle, so that ethylbenzene accumulates. If the ethylbenzene is removed by some way in order to prevent the accumulation thereof, ethylbenzene in an amount corresponding to the degree of removal thereof circulates in the "separation-isomerization cycle". If the amount of the circulating ethylbenzene is decreased, the total amount of the circulating materials is also decreased, so that the utility consumption after the p-xylene-separation step is decreased, which is greatly advantageous from the economical view point. In other words, when the amounts of the circulating materials are equal, increase in the production of *p*-xylene may be attained to a degree corresponding to the decrease in the concentration of ethylbenzene.

There are two usual methods for removing ethylbenzene. One is the reforming method in which ethylbenzene is isomerized to xylene simultaneously with the isomerization of xylene in the isomerization step. Another is the dealkylation method in which ethylbenzene is converted to benzene by hydrogenation and dealkylation thereof in the isomerization step of xylene, and then the benzene is separated by distillation in the subsequent distillation separation step. However, by the isomerization method, the degree of conversion of ethylbenzene is about 20 to 30% at most due to the equilibrium between ethylbenzene and xylene. In contrast, since the dealkylation reaction is a substantially non-equilibrium reaction, the degree of conversion of ethylbenzene may be made high by the dealkylation method. Therefore, at present, ethylbenzene is usually removed by the dealkylation method. However, even if the system is operated at a very high degree of conversion of ethylbenzene in the isomerization step and even if ethylbenzene is removed at a level as high as possible, since the C8 aromatic hydrocarbon mixed feedstocks supplied to the "separation-isomerization cycle" intrinsically contains ethylbenzene, the amount of ethylbenzene to be supplied to the p-xylene-separation step cannot be decreased to a level lower than the content of this intrinsically contained ethylbenzene.

Patent Literatures 1 and 2 disclose methods in which the amount of ethylbenzene supplied to the "separation-isomerization cycle" is decreased by preliminarily deethylating most of the ethylbenzene contained in the fresh feedstocks to convert it to benzene in one path and separating the benzene by distillation, in order to reduce the amount of the ethylbenzene supplied to the "separation-isomerization cycle" to almost zero. However, in the methods concretely described therein, it is necessary to eliminate high boiling components by distillation separation before the dealkylation reaction in order to decrease the concentration of the high boiling components having carbon atoms of not less than 9 to prevent decrease in catalyst activity.

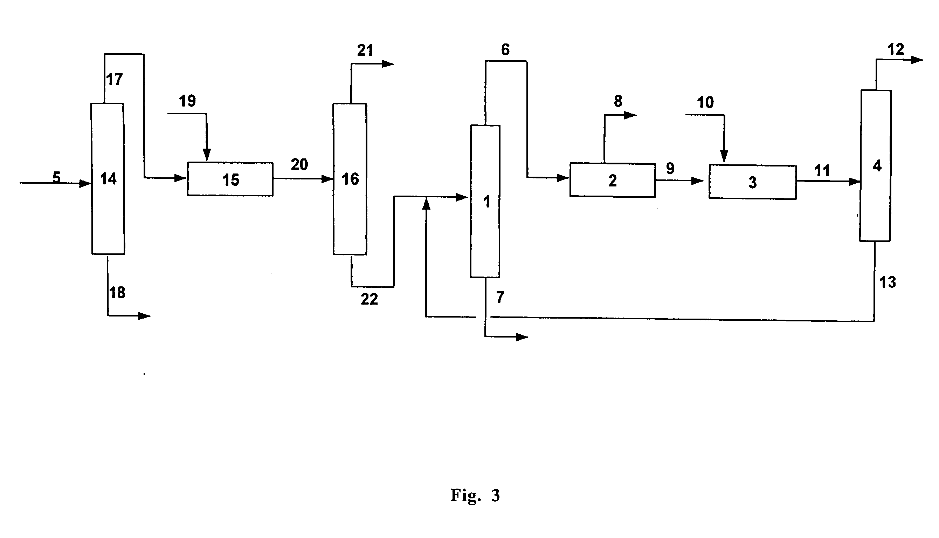

Fig. 3 is a flow chart showing a mode of the methods. A deethylation/xylene-isomerization step 15 by which ethylbenzene is preliminarily deethylated in one path, a low boiling point components-distillation separation step 16 by which the benzene generated by the deethylation of ethylbenzene is recovered by distillation separation, and a high boiling components-distillation separation step 14 by which the high boiling components intrinsically contained in the C8 aromatic hydrocarbon mixed feedstocks, are newly added. That is, to protect the catalyst used in the deethylation step, high boiling components contained in the C8 aromatic hydrocarbon mixed feedstocks supplied through the line denoted by stream 5, which act as a catalyst poison, are separated by distillation through the line denoted by stream 18, and the distillate fraction was transferred to the deethylation/xylene-isomerization step 15 through the line denoted by stream 17. To the deethylation step, hydrogen or a hydrogen-containing gas is also transferred through the line denoted by stream 19. The C8 aromatic hydrocarbon mixed feedstocks in which ethylbenzene has been highly deethylated and which contains byproducts is transferred to the low boiling point components-distillation separation step 16 through the line denoted by stream 20. The low boiling components such as benzene and toluene byproduced in the deethylation step is separated and eliminated through the line denoted by stream 21, and the high boiling components are transferred to the above-described "separation-isomerization" cycle through the line denoted by stream 22. However, in this case, since the high boiling components-distillation separation step 14 and the low boiling point components-distillation separation step 16 are newly incorporated, the utility consumption is increased, so that there is a problem in that the advantageous merit introducing the steps is decreased.

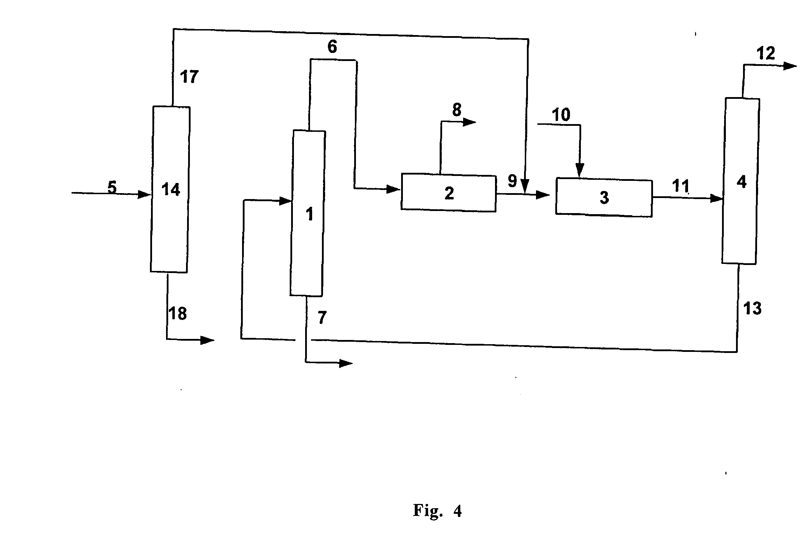

With the above-described mode, needless to say, the costs for grass roots for the deethylation step are additionally necessary. Thus, a process for producing *p*-xylene is disclosed in Patent Literature 4, in which a catalyst having a function to highly deethylate ethylbenzene in addition to the usual function to isomerize xylene is introduced into the xylene-isomerization step in the original "separation-isomerization" cycle. In this method, as shown in Fig. 4, a fresh feedstocks denoted by stream 17 is mixed with raffinate xylene from the p-xylene-separation step, denoted by stream 9, and the mixture is directly supplied to the isomerization step 3 to deethylate ethylbenzene at a high degree of conversion, followed by elimination of the low boiling components containing benzene in the low boiling components-distillation separation step 4. Thereafter, the recycled material denoted by stream 13, which has a very low ethylbenzene concentration and a high *p*-xylene concentration is transferred to the high boiling components-distillation separation step 1 and to the *p*-xylene-separation step 2. This method is called direct feed method. By this method, increase in the production of *p*-xylene may be attained with a relatively small equipment investment, because the method may be carried out by a simple modification of equipment such as increasing the catalyst used in the xylene-isomerization step or replacing the catalyst with a catalyst having a high activity, without independently building an equipment for the deethylation step. However, it is described in Patent Literature 4 that in cases where the high boiling components having not less than 9 carbon atoms are contained in the fresh feedstocks, the high boiling components must be preliminarily eliminated by distillation separation in order to prevent the decrease in the catalyst activity. Therefore, even with this method, installation of the high boiling components-distillation separation step 14 is necessary, so that the equipment investment and increase in the utility consumption due to the incorporation of this step are inevitable.

In general, to highly convert ethylbenzene, the reaction temperature is raised or the material is brought into contact with a catalyst having a high activity. As the degree of conversion of ethylbenzene increases, the decrease in the yield of xylene is actualized. The losses are constituted mainly by (1) the loss that xylene is converted to toluene by transalkylation reaction between benzene and xylene, which benzene is generated by the deethylation of ethylbenzene. Other losses are (2) the loss that xylene is converted to toluene and trimethylbenzene by disproportionation reaction between xylene; and (3) the loss that xylene is converted to non-aromatics such as cycloparaffins, *n*-paraffins and *iso*-paraffins by nuclear hydrogenation of xylene. Further, since the benzene obtained by distillation separation is contaminated with the non-aromatics generated by the nuclear hydrogenation reaction, an extraction treatment such as sulfolane unit is additionally necessary, which brings about an economic disadvantage that the utility consumption is increased due to the increase in the amount of treatment in the extraction step.

Patent Literature 3 discloses to use a material containing C9 and C10 aromatic hydrocarbons which are high boiling components and to convert ethylbenzene in the xylene-isomerization step, but it does not disclose to use ethyltoluene as the C9+ aromatic hydrocarbons and to convert it. More particularly, in an Example of Patent Literature 3, it is described that conversion of ethylbenzene was carried out using a feed containing C9+ aromatic hydrocarbons in an amount of less than 0.01 % by weight, and that as a result of this reaction, reaction products containing C9+ aromatic hydrocarbons in an amount of 0.1 % by weight and 0.2% by weight, respectively, were obtained. According to the explanation in this literature about the side reactions in which the C9+ aromatic hydrocarbons are involved, the increase in the amount of the C9+ aromatic hydrocarbons in the above-described reaction is thought to be caused by the side reactions such as the generation of trimethylbenzene and diethylbenzene by the disproportionation reactions of xylene and ethylbenzene, and generation of methylethylbenzene and dimethylethylbenzene by the transalkylation reaction of ethylbenzene and xylene. Patent Literature 3 further discloses, as an optional case, a method in which toluene is intentionally mixed so as to reduce the loss of xylene due to the disproportionation reaction between xylene. However, since the intentionally mixed toluene is a valuable and important basic material used in many applications, that is, used as the base of gasoline with a high octane value, as a solvent, as a material for disproportionation step, and so on, toluene is a component which is not desired to be added to the material if at all possible, if there is another method to reduce the loss of xylene.

Patent Literature 1: JP-A-1-56626

Patent Literature 2: US-B-6,342,649

Patent Literature 3: US-B-5,977,429

Patent Literature 4: JP-A-8-143483

Disclosure of the Invention

Problems Which the Invention Tries to Solve

An object of the present invention is to provide a process for converting ethylbenzene, by which ethylbenzene in a material containing C8 aromatic hydrocarbons is converted to benzene at a high degree of conversion.

Another object of the present invention is to provide an economically advantageous process by which the degree of conversion of ethylbenzene is high and loss of xylene is small when the ethylbenzene in the material containing C8 aromatic hydrocarbons is converted to benzene and simultaneously xylene is isomerized.

Still another object of the present invention is to provide a method for recovering benzene with a high purity from the reaction product after the reaction of converting ethylbenzene in the material containing C8 aromatic hydrocarbons and isomerizing xylene.

Means for Solving the Problems

The present inventors intensively studied to discover that the above-described objects may be attained by making the feedstocks contain ethyltoluene and by bringing the feedstocks into contact with a strong acid type catalyst highly resistant to catalyst poisons, in the presence of $H_2$, thereby reaching the present invention.

That is, the present invention has the following constitution:

(1) A process for converting ethylbenzene, comprising the step of bringing a C8 aromatic hydrocarbon mixed feedstocks containing ethylbenzene into contact with an acid type catalyst(s) containing at least one metal selected from the group consisting of the metals belonging to Group VII and Group VIII in the presence of $H_2$ to convert the ethylbenzene to benzene, the feedstocks containing C9-C10 aromatic hydrocarbons including ethyltoluene, the ethyltoluene being converted to toluene together with the conversion of ethylbenzene.

(2) The process according to the above-described (1), further comprising separating, by distillation, the benzene produced by the reaction, and recovering the benzene at a purity of not less than 99.8% by weight.

(3) A process for producing p-xylene, comprising the steps of:

subjecting a C8 aromatic hydrocarbon mixed feedstocks containing ethylbenzene and xylene to the process according to the above-described (1) or (2) to isomerize the xylene together with converting the ethylbenzene to benzene; and

separating p-xylene from the obtained reaction product.

(4) A process for producing p-xylene, comprising:

a first deethylation/xylene-isomerization step of subjecting a C8 aromatic hydrocarbon mixed feedstocks containing ethylbenzene and xylene to the process according to the above-described (3) to isomerize the xylene together with converting the ethylbenzene to benzene;

a step of separating p-xylene from the reaction product obtained in the first deethylation/xylene-isomerization step;

a step of subjecting xylene contained in the residue after separation to a second xylene-isomerization step to isomerize the xylene; and

separating p-xylene again from the reaction product of the second xylene-isomerization step.

(5) A process for producing p-xylene, comprising:

a deethylation/xylene-isomerization step of subjecting a C8 aromatic hydrocarbon mixed feedstocks containing ethylbenzene and xylene to the process according to the above-described (3) to isomerize the xylene together with converting the ethylbenzene to benzene;

a step of separating p-xylene from the reaction product obtained in the deethylation/xylene-isomerization step; and

a step of recycling the residue after separation obtained in the separation step by supplying the residue to the deethylation/xylene-isomerization step.

(6) A process for producing p-xylene by using a p-xylene-producing equipment for carrying out the first deethylation/xylene-isomerization step, p-xylene-separation step and second xylene-isomerization step recited in the above-described process (4), wherein the equipment comprises a bypass line which does not pass through the first deethylation/xylene-isomerization step, and wherein the C8 aromatic hydrocarbon mixed feedstocks mixed with the C9-C 10 aromatic hydrocarbons containing ethyltoluene is supplied to the p-xylene-separation step, as required.

Effects of the Invention

By the present invention, xylene loss may be reduced and ethylbenzene may be highly hydrogenated and deethylated to be converted to benzene, by carrying out the isomerization step converting ethylbenzene to benzene and simultaneously isomerizing xylene, wherein C9, C10 aromatic hydrocarbons containing ethyltoluene are mixed with the C8 aromatic hydrocarbon mixed feedstocks containing ethylbenzene, and wherein the resulting feed are contacted with an acid type catalyst(s) containing at least one metal selected from the group consisting of the metals belonging to Group VII and Group VIII in the presence of $H_2$. Further, the ethyltoluene in the feedstocks is converted to useful toluene by deethylation and the toluene may be recovered as a final by-product.

Further, by the present invention, benzene with a high purity may be recovered from the reaction product generated by the conversion of ethylbenzene in the feedstocks containing C8 aromatic hydrocarbons. By virtue of this, benzene may be obtained as a final product after recovery without an extraction step.

Brief Description of the Drawings

Fig. 1 is a conceptual diagram showing the flow when a bypass line which does not pass through the first deethylation/xylene-isomerization step, in a p-xylene-producing equipment for carrying out the first deethylation/xylene-isomerization step, the p-xylene-separation step and the second xylene-isomerization step.

Fig. 2 is a conceptual diagram showing the flow of "separation-isomerization cycle" for usual p-xylene production, in which the deethylation step is not introduced.

Fig. 3 is a conceptual diagram showing the flow of "separation-isomerization cycle" for usual *p*-xylene production, in which the deethylation step is introduced.

Fig. 4 is a conceptual diagram showing the flow of a usual direct feed method in which a deethylation step is not introduced, the fresh feedstocks is mixed with raffinate xylene, and the obtained mixed material is transferred to a xylene-isomerization step which has a function to highly deethylate the ethylbenzene.

Best Mode for Carrying out the Invention

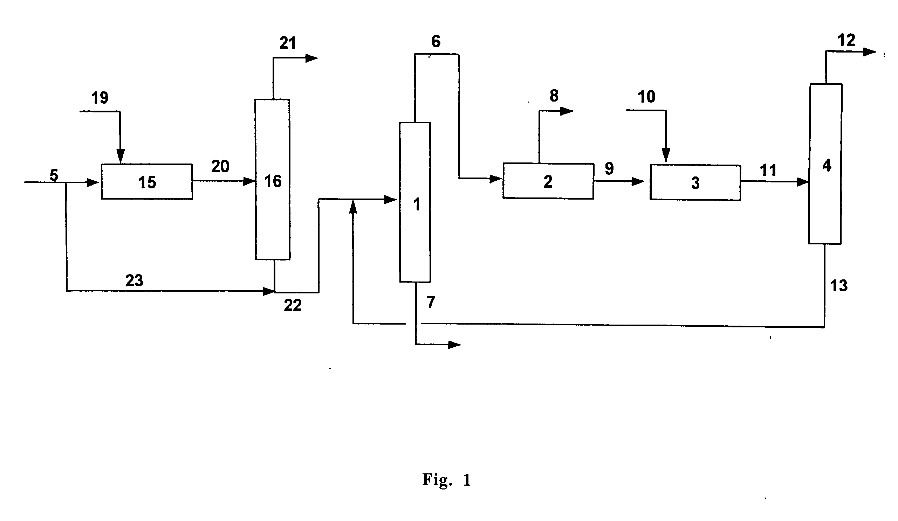

The present invention is characterized in that when carrying out deethylation reaction of ethylbenzene, the C8 aromatic hydrocarbon mixed feedstocks containing ethylbenzene to be supplied contains ethyltoluene. Usually, ethyltoluene is contained in the high boiling components in an amount of 5 to 15% by weight in the feedstocks containing C8 aromatic hydrocarbons, which feedstocks is supplied to the "separation-isomerization cycle". Therefore, the distillation tower for separation of the high boiling components by distillation when conducting the deethylation step may be omitted. Fig. 1 shows a preferred mode of the flow for carrying out the present invention, and the high boiling components-distillation separation step 14 shown in Figs. 3 and Fig. 4 showing the flow of the prior art may be omitted.

That is, according to the present invention, when carrying out the deethylation reaction of ethylbenzene in the feedstocks supplied to the deethylation step, deethylation reaction of the ethyltoluene contained in the supplied feedstocks containing C9 and C10 aromatic hydrocarbons is simultaneously carried out to convert it to toluene, the ethyltoluene may be usefully utilized to obtain toluene. Thus, unlike the prior art represented by Patent Literature 3, it is not necessary to additionally mix useful toluene having many other uses. Further, since the disproportionation reaction of xylene and the transalkylation reaction between benzene generated by deethylation of ethylbenzene and xylene, which reactions are the causes of the above-described xylene loss, are equilibrium reactions, these side reactions are reduced by the existence of toluene obtained in the deethylation of ethyltoluene, so that the xylene loss is decreased.

The catalyst used in the process of the present invention is an acid type catalyst obtained by doping a solid acid with a prescribed metal(s) described below. As the solid acid, acid type zeolites are exemplified. Among the acid type zeolites, those which may be used in the present invention include pentasil zeolites such as the pentasil (MFI type) zeolite having pores of 10-membered oxygen ring (for example, see Example 1 on pages 4-5 of JP-B-60-35284 and Example 1 on page 7 of JP-B-46-10064). As the zeolite, either naturally occurring zeolites or synthetic zeolites may be employed, and synthetic zeolites are preferred. Such a pentasil zeolite *per se* as well as its production process is well-known, and an example of the synthetic process is concretely described in the Examples below. The catalytic performance of zeolites varies depending on the composition, especially on the silica/alumina molar ratio ($SiO_2/Al_2O_3$ molar ratio) and on the size of the crystallite thereof, even when the zeolite structure is the same.

The preferred range of the silica/alumina molar ratio of the zeolite varies also depending on the structure of the zeolite. For example, in case of a synthetic pentasil zeolite, the preferred silica/alumina molar ratio is 10 to 70, more preferably 20 to 55. This molar ratio may be attained by controlling the ratio of the components when synthesizing the zeolite. Further, by removing aluminum constituting the zeolite structure with an aqueous acid solution such as hydrochloric acid or with an aluminum-chelating agent such as ethylenediaminetetraacetic acid (EDTA), the silica/alumina molar ratio may be increased. Conversely, by treating the zeolite with aqueous aluminum nitrate solution, aqueous sodium aluminate solution or the like, aluminum may be introduced into the zeolite structure to decrease the silica/alumina molar ratio of the zeolite to attain the preferred silica/alumina molar ratio. The silica/alumina molar ratio may be easily determined by atomic absorption spectrometry, fluorescent X-ray diffraction method, ICP (inductively coupled plasma) spectrometry or the like.

Since the synthetic zeolites are generally in the form of powder, it is preferred to mold the zeolite. Examples of the molding methods include compression molding method, roll molding method and extrusion method. Among these molding methods, extrusion method is preferred. In the extrusion method, a binder(s) such as alumina sol, alumina gel, bentonite and/or kaolin, as well as a surfactant(s) such as sodium dodecylbenzene sulfonate, Span (trademark) and/or Tween (trademark), is(are) added as required as a molding aid(s), and kneaded with the powder.

As required, a machine such as a kneader is used. Further, depending on the metal(s) to be added to the catalyst, a metal oxide(s) such as alumina, titania and/or the like is(are) added when molding the zeolite to increase the amount of the metal(s) carried by the catalyst and/or to promote dispersion. The kneaded product is extruded through a screen. Industrially, an extruder is used. The kneaded product extruded through a screen is in the form of noodle. The size of the molded product is determined by the pore size of the screen. A pore size of the screen of 0.2 to 1.5mm diameter is preferably employed. The molded product in the form of noodle extruded through the screen may preferably be treated with a Marumelyzer (trademark) to round off the edges. The thus prepared molded product is preferably dried at 50°C to 250°C. After drying, the molded product is preferably baked at 250°C to 600°C, more preferably at 350°C to 600°C.

The thus prepared molded product is then subjected to ion-exchange treatment for giving solid acidity. Examples of the method for giving solid acidity include a method in which the molded product is subjected to ion-exchange treatment with a compound(s) containing ammonium ion (e.g., $NH_4Cl$, $NH_4NO_3$, $(NH_4)_2SO_4$ and the like) to introduce $NH_4$ ions into the ion-exchange sites in the zeolite, and then the $NH_4$ ions are exchanged with hydrogen ions by drying and baking the zeolite; and a method in which hydrogen ions are directly introduced into the ion-exchange sites of zeolite by treating the zeolite with a compound(s) containing an acid (e.g., HCl, $HNO_3$, $H_3PO_4$ and the like). Since the latter method may break the zeolite structure, the former method is preferred, that is, the zeolite is preferably treated with an ammonium ion-containing compound(s). Alternatively, solid acidity may be added by introducing divalent and/or trivalent metal ions into the ion-exchange sites of zeolite. Examples of the divalent metal ion include $Mg^{2+}$, $Ca^{2+}$, $Sr^{2+}$ and $Ba^{2+}$. Examples of the trivalent metal ion include rare earth metal ions such as $Ce^{3+}$, $La^{3+}$ and the like. The method in which the divalent and/or trivalent metal ions are introduced and the method in which ammonium ions are introduced or hydrogen ions are directly introduced may be employed in combination, and this combination may be more preferred in some cases. The ion-exchange treatment is carried out by a batch process or a flow method in which the carrier such as zeolite is treated with a solution containing the ions, usually with an aqueous solution. The treatment temperature is usually between room temperature and 100°C.

After the ion-exchange treatment, at least one metal selected from the group consisting of the metals belonging to Group VII and Group VIII is carried. By making $H_2$ exist in the catalytic reaction system and by making the catalyst carry a hydrogenating active metal(s), deterioration of the catalyst with time may be prevented. Preferred examples of the hydrogenating active metal include platinum, palladium, rhenium and the like. The preferred amount of the metal to be carried varies depending on the metal. For example, in case of platinum, the preferred amount is 0.005 to 0.5% by weight, more preferably 0.01 to 0.3% by weight. In case of palladium, the preferred amount is 0.05 to 1% by weight. In case of rhenium, the preferred amount is 0.01 to 5.0% by weight, more preferably 0.1 to 2% by weight. A large amount of the carried hydrogenating metal is not preferred because aromatic hydrocarbons are nuclear-hydrogenated. On the other hand, if the amount of the carried hydrogenating metal is too small, the supply of hydrogen in the deethylation reaction is not sufficient, so that deterioration of catalyst is brought about. Thus, the type of the metal(s) to be selected and combination thereof, as well as the amount of the metal(s) should be appropriately adjusted depending on the target performance. The method for carrying the metal(s) comprises immersing the catalyst in a solution, usually aqueous solution, containing at least one of platinum, palladium and rhenium. As the compound(s) containing platinum component, chloroplatinic acid, ammonium chloroplatinate and/or the like may be employed. As the compound(s) containing palladium component, palladium acetate, palladium acetylacetonate, palladium chloride, palladium nitrate and/or the like may be employed. As the compound(s) containing rhenium component, perrhenic acid, ammonium perrhenate and/or the like may be employed.

The thus prepared catalyst is preferably dried at 50°C to 250°C for not less than 30 minutes, and is preferably baked at 350°C to 600°C for not less than 30 minutes before use.

As the catalyst, one type of catalyst may be used individually, or two or more types of catalyst may be used in combination.

The catalytic reaction using the catalyst prepared as described above may be carried out by various reaction operations which *per se* are well-known in the art. The reaction may be carried out by any of fixed-bed process, moving bed process and fluidized bed process. Among these processes, fixed-bed process is especially preferred because of ease of operation. In these reaction processes, the catalyst may be used under the following reaction conditions: That is, the reaction temperature is 200°C to 500°C, preferably 250°C to 450°C. The reaction pressure is from atmospheric pressure to 10 MPa, preferably 0.3 to 2 MPa. The weight hourly spatial velocity (WHSV) which expresses the contact time is 0.1 to 50 $hr^{-1}$, preferably 0.5 to 20.0 $hr^{-1}$. The reaction is carried out in the presence of $H_2$, and the molar ratio of $H_2$ to the feed oil is 0.5 to 10 mol/mol, preferably 1.5 to 5.0 mol/mol. $H_2$ may be made to exist in the reaction system by introducing hydrogen gas into the reaction system. The feed oil may be in the state of either liquid or gas.

The ethyltoluene which is made to be contained in the feed oil may be any of p-ethyltoluene, *m*-ethyltoluene and o-ethyltoluene, or may be a mixture of the isomers. The total amount of these ethyltoluenes contained in the feed material is preferably not less than 1% by weight, more preferably not less than 3% by weight, still more preferably not less than 5% by weight. As for the upper limit, the amount is usually preferred to be not more than 20% by weight, more preferably not more than 15% by weight. As mentioned above, since the C8 aromatic hydrocarbons obtained from naphtha by reforming treatment or fractional distillation contain ethyltoluene, this ethyltoluene may be used as it is. Therefore, installation of the distillation tower for removing high boiling components, which was necessary in the conventional processes, may be omitted.

The ethyltoluene may be made to be contained by adding ethyltoluene to the feedstocks. In cases where ethyltoluene is added to the feedstocks, the ethyltoluene may be added individually, or a mixture of ethyltoluene and other C9-C10 aromatic hydrocarbons may be added to the feed material.

By the process according to the present invention which uses the above-described acid type catalyst, since the ethyltoluene contained in the feedstocks may be deethylated at a degree of conversion of not less than 50% by weight, in a more preferred mode, at not less than 70% by weight, and in an especially preferred mode, at not less than 80% by weight, much toluene useful for the reduction of xylene loss may be obtained even if a feedstocks having a low ethyltoluene concentration is used.

Further, the benzene byproduced by the dealkylation reaction of ethylbenzene is usually purified by distillation separation and by extraction separation such as sulfolane step. However, in cases where the above-described acid type catalyst is used, since the generation of non-aromatic components such as cyclohexane, methylcyclopentane, n-hexane and the like, which have boiling points relatively close to that of benzene and so are difficult to be separated by distillation is small, benzene with a high purity may be obtained only by distillation separation omitting the extraction treatment.

An equation for estimating the purity of the benzene as a final product separated from the generated reaction product liquid composition by distillation is, for example, described in JP-A-2002-504946, which is described below. In the present invention, the term "purity of benzene" means the purity of benzene calculated by this equation for estimating the purity of benzene as a final product.

$$\text{Estimated Purity of Final product Benzene} = ([\text{benzene concentration}]/a+b+c+d+[\text{benzene concentration}]) * 100(\%))$$

wherein a to d represent the following:

a = 0.1 * [$n$-C6 paraffin concentration]

b = 0.7 * [methylcyclopentane concentration]

c = 1.0 * [cyclohexane concentration]

d = 1.0 * [C7 naphthene paraffin concentration]

In cases where rhenium is used as the metal to be contained in the acid type catalyst, since the hydrogenation ability thereof is relatively mild and so the loss of aromatic compounds due to the decomposition by nuclear hydrogenation is small, generation of the above-described impurities having the boiling points relatively close to that of benzene is small. As a result, the estimated purity of the final product benzene is not less than 99.8% by weight. Thus, the benzene is obtained as a product of chemical grade by the distillation separation without a further purification such as extraction step. On the other hand, the toluene generated by the dealkylation reaction of ethyltoluene is recovered by distillation operation, and may be usefully used as a base of gasoline, a solvent or as a material for producing benzene by disproportionation step.

By the present invention, not only the ethylbenzene is converted to benzene by carrying out the above-described conversion process of ethylbenzene using the C8 aromatic hydrocarbon mixed feedstocks containing ethylbenzene and xylene, but also xylene may be isomerized (the step of converting ethylbenzene to benzene and isomerizing xylene is referred to as "deethylation/xylene-isomerization step" or "first deethylation/xylene-isomerization step". By this, a reaction product in which not only the degree of conversion of ethylbenzene is high, but also the xylene loss is small may be obtained. By separating *p*-xylene from this reaction product, *p*-xylene may be obtained. The separation of *p*-xylene from the reaction product may be carried out by the methods which *per se* are well-known, for example, by the cryogenic separation or adsorptive separation utilizing the difference in degree of adsorption by zeolite adsorbent.

Further, in the present invention, raffinate xylene which is a separation residue after separation of *p*-xylene as described above, which has a low *p*-xylene concentration may be subjected to isomerization of xylene by providing a second xylene-isomerization step. The method for isomerization conducted in this second xylene-isomerization step is not restricted, and the isomerization of xylene may be carried out by an ordinary process, or may be carried out by a step similar to the above-described "deethylation/xylene-isomerization step". From the reaction product obtained in this second xylene-isomerization step, *p*-xylene may be separated again. The second residue after separation of *p*-xylene may be recycled by mixing it with the C8 aromatic hydrocarbon mixed feedstocks supplied to the first deethylation/xylene-isomerization step, or may recycled by being supplied to the second xylene-isomerization step together with the first residue after separation, thereby forming a "separation-isomerization cycle".

Further, in the present invention, *p*-xylene may be produced by conducting the deethylation/xylene-isomerization step in which ethylbenzene is converted and xylene is isomerized, using a C8 aromatic hydrocarbon mixed feedstocks containing ethylbenzene and xylene, which deethylation/xylene-isomerization step is similar to the above-described first deethylation/xylene-isomerization step, separating *p*-xylene from the obtained reaction product, and by recycling the residue after separation by supplying the residue to the above-described deethylation/xylene-isomerization step. This process may also be utilized in the above-described direct feed process. That is, *p*-xylene may be separated again by mixing the C8 aromatic hydrocarbon mixed feedstocks containing ethyltoluene obtained omitting the distillation separation of the high boiling components with the raffinate xylene after separating *p*-xylene, transferring the mixture to the xylene-isomerization step in which the above-described acid type catalyst is introduced to deethylate the ethylbenzene and to isomerize the xylene, and by separating *p*-xylene again from the thus obtained reaction product. That is, in cases where the above-described acid type catalyst is introduced in the isomerization step, the high boiling components-distillation separation step 14 may be omitted in Fig. 4 showing a conceptual diagram of a usual direct feed method.

In cases where the above-described first deethylation/xylene-isomerization step, *p*-xylene-separation step and the second xylene-isomerizing step are carried out, and in cases where the "separation-isomerization cycle" is further added thereto, it is preferred to provide a bypass line such as a bypass line 23 of deethylation/xylene-isomerization step as shown in Fig. 1 in the *p*-xylene-producing equipment for conducting these steps.

This is because that even when the deethylation reaction step is stopped for periodic maintenance or stopped by emergency shutdown, it is not necessary to stop the entire subsequent "separation-isomerization cycle" by changing the supply route of the C8 aromatic hydrocarbon mixed feedstocks to the route through the bypass line, so that the decrease in the production of *p*-xylene may be reduced as small as possible. Further, in cases where the above-described deethylation/xylene-isomerization step is used as the xylene-isomerization step, since the conversion of ethylbenzene contained in the C8 aromatic hydrocarbon mixed feedstocks is simultaneously conducted, ethylbenzene may be converted at a high degree of conversion in spite of the fact that the first deethylation/xylene-isomerization step is omitted, and moreover, since the xylene loss is small and isomerization of xylene may be carried out, the influence by the omission of the first deethylation/xylene-isomerization step may be reduced as small as possible.

Examples

1. Synthesis of Pentasil Zeolite

In 698.6 g of water, 54.2 g of aqueous sodium hydroxide solution (NaOH content: 48.0% by weight, $H_2O$ content: 52.0% by weight, Toagosei Co., Ltd.) and 16.6 g of tartaric acid powder (tartaric acid content: 99.7% by weight, $H_2O$ content: 0.3% by weight, CaHC Co., Ltd.) were dissolved. To this solution, 9.9 g of sodium aluminate solution ($Al_2O_3$ content: 13.4% by weight, $Na_2O$ content: 13.8% by weight, $H_2O$ content: 43.9% by weight, Sumitomo Chemical Co., Ltd.) was added and the mixture was made to be a uniform solution. To this mixed solution, 111.5 g of hydrated silisic acid ($SiO_2$ content: 89.4% by weight, $Al_2O_3$ content: 2.4% by weight, $Na_2O$ content: 1.6% by weight, Nipseal VN-3, Nihon Silica Co., Ltd.) was slowly added with stirring to prepare an aqueous reaction mixture in the form of uniform slurry. The composition ratio (molar ratio) of this reaction mixture was as follows:

$SiO_2/Al_2O_3$: 77
OH-/$SiO_2$ :0.3002
A/$Al_2O_3$ :5.14 (A: tartaric acid salt)
$H_2O/SiO_2$: 25

The reaction mixture was placed in a 1000 ml-autoclave and the autoclave was sealed, followed by allowing the reaction at 160°C for 72 hours with stirring at 250 rpm. After the reaction, washing of the reaction product with distilled water and subsequent filtration were repeated 5 times, and the resulting product was dried overnight at about 120°C.

Analysis of the obtained product with an X-ray diffraction apparatus using Cu vessel and K$\alpha$-ray revealed that the obtained product was a pentasil zeolite.

Fluorescent X-ray diffraction analysis of this pentasil zeolite revealed that the silica/alumina molar ratio thereof was 49.0.

2. Production of Catalyst

(1) Production of Catalyst A (use of Catalyst A is outside the scope of the present invention)

To the thus synthesized pentasil zeolite in an amount of 10 g in terms of the absolute dryness standard (calculated from the loss on ignition after baking at 500°C for 20 minutes), hydrated alumina (produced by Sumitomo Chemical Co., Ltd.) having pseudoboehmite structure in an amount of 30 g in terms of the absolute dryness standard, and 60 g of alumina sol ($Al_2O_3$ content: 10% by weight, produced by Nissan Chemical Industries, Ltd.) were added and the mixture was sufficiently mixed, followed by drying the mixture in a dryer at 120°C until the mixture became a liquid in the form of clay. The obtained kneaded mixture was extruded through a screen having a pore diameter of 1.2 mm. The extruded molded product was dried overnight at 120°C. Thereafter, the temperature was slowly raised from 350°C to 540°C, and the product was baked at 540°C for 2 hours. Twenty grams of this molded product of pentasil zeolite was placed in an aqueous solution containing 11 parts by weight of $NH_4Cl$ and 5 parts by weight of $CaCl_2$ per 100 parts by weight, under the absolute dryness standard, of the pentasil zeolite molded product, and the solid-liquid ratio was adjusted to 2.0 kg/L with pure water, followed by incubating the mixture at 80°C for 1 hour. The molded product was then washed 6 times with pure water batchwise. The thus obtained ion-exchanged pentasil zeolite molded product was dried overnight at 120°C. Before using the product in a catalytic reaction, the catalyst was treated with hydrogen sulfide gas flow at 250°C for 2 hours, and then baked at 540°C for 2 hours in the air to obtain Catalyst A.

(2) Production of Catalyst B

A molded product containing pentasil (MFI) zeolite was produced and ion-exchange with ammonium ions and calcium ions was carried out in the same manner as in the production of Catalyst A. Twenty grams of the ion-exchanged and dried pentasil zeolite molded product after the ion-exchange was immersed in 40 ml of an aqueous solution of perrhenic acid containing 80 mg of Re in terms of Re at room temperature, and the resulting mixture was left to stand for 2 hours. The mixture was stirred at intervals of every 30 minutes, and after draining liquid, the product was dried overnight at 120°C. Before using the product in a catalytic reaction, the catalyst was treated with hydrogen sulfide gas flow at 250°C for 2 hours, and then baked at 540°C for 2 hours in the air to obtain Catalyst B. Analysis of the Re carried in Catalyst B by ICP spectrometry revealed that the amount of the Re was 2010 ppm by weight in terms of Re.

Example 1, Comparative Example 1

The above-described Catalysts A and B were packed in reaction tubes, respectively, and reaction tests were performed. The compositions of the 4 types of feed materials used are shown in Table 1 below. The analysis of the compositions of the feed materials and the reaction products were carried out using 3 gas chromatography apparatuses equipped with a hydrogen flame detector. The separation columns were as follows:

(1) Gas components (components from methane to n-butane in gas):

Medium: Unipak S (trademark), 100-150 mesh
Column: made of stainless steel; length: 4 m; inner diameter: 3 mm
$N_2$: 1.65kg/cm$^2$-G
Temperature: 80°C

(2) Components in liquid, with boiling points close to that of benzene (from methane to n-butane dissolved in the liquid and from 2-methyl-butane to benzene which are liquid components):

Medium: 25% polyethylene glycol 20M/carrier "Shimalite" 60-80 mesh
Column: made of stainless steel; length: 12 m; inner diameter: 3 mm
$N_2$:2.25kg/cm$^2$-G
Temperature: from 68°C to 180°C at a raising rate of 2°C/min

(3) Components with higher boiling points than liquid benzene (from benzene to heavy end components)

Spelco wax fused silica capillary: length: 60 m; inner diameter: 0.32 mm; film
thickness: 0.5 μm
He linear velocity: 23 cm/sec.
Temperature: from 67°C at a raising rate of 1°C/min, and from 80°C to 200°C at a raising rate of 2°C/min.

Table 1

| Compositions of Feed Materials (Unit:% by weight) | | | | | |
|---|---|---|---|---|---|
| | Material A | Material B | Material C | Material D | Material E |
| EB | 16.2 | 16.0 | 15.5 | 15.0 | 6.5 |
| PX | 20.0 | 19.7 | 19.1 | 18.5 | 4.8 |
| MX | 44.1 | 43.4 | 42.1 | 40.8 | 51.5 |
| OX | 19.7 | 19.4 | 18.8 | 18.2 | 25.0 |
| ET | 0.0 | 1.0 | 3.0 | 5.0 | 4.8 |

(continued)

| Compositions of Feed Materials (Unit:% by weight) | | | | | |
|---|---|---|---|---|---|
| | Material A | Material B | Material C | Material D | Material E |
| Other C9+ | 0.0 | 0.5 | 1.5 | 2.5 | 7.4 |
| Total | 100.0 | 100.0 | 100.0 | 100.0 | 100.0 |

"EB" denotes ethylbenzene, "PX" denotes *p*-xylene, "MX" denotes *m*-xylene, "OX" denotes o-xylene and "ET" denotes ethyltoluene. "C9+" denotes compounds having not less than 9 carbon atoms. Materials A to D are simulations of the feedstocks to be supplied to the deethylation step before the "separation-isomerization" cycle. Material E is a simulation of C8 aromatic hydrocarbon mixed feedstocks containing ethyltoluene and compounds having not less than 9 carbon atoms for the direct feed process.

In a reaction tube, 7.5 g of Catalyst A or Catalyst B was packed and the above-described feed oils were allowed to react under the following conditions:

Reaction Conditions:
WHSV(hr$^{-1}$): 4.2
Reaction Temperature (°C): 405
Reaction Pressure (MPa): 0.9
H$_2$/Feed (mol/mol): 3.5

Test results are shown in Table 2.

Table 2

| | | | | | (Unit: % by weight) |
|---|---|---|---|---|---|
| | Comparative Example 1 | | Example 1 | | |
| | Comparative Example 1-A | Comparative Example 1-B | Example 1-C | Example 1-D | Example 1-E |
| Catalyst used | Catalyst A | Catalyst B | Catalyst B | Catalyst B | Catalyst B |
| Feed material used | Material A | Material A | Material B | Material C | Material D |
| Degree of conversion of EB (%) | 50.0 | 90.0 | 90.0 | 90.0 | 90.0 |
| PX/(PX+MX+OX) | 20.5 | 23.5 | 23.5 | 23.5 | 23.5 |
| ET concentration in reaction product | 0.0 | 0.1 | 0.2 | 0.6 | 1.0 |
| Toluene (TOL) concentration in reaction product | 0.7 | 1.5 | 2.1 | 3.3 | 4.5 |
| Degree of conversion of ET | - | - | 80.5 | 80.8 | 81.0 |
| (PX+MX+OX) Yield | 98.8 | 96.6 | 96.8 | 96.9 | 97.1 |

From the results of Example 1, it can be seen that the yield of xylene was increased by treating the feedstocks containing ethyltoluene using the acid type zeolite catalyst carrying rhenium which is a hydrogenating active metal. This is thought to be because that toluene is produced by dealkylation of ethyltoluene and this toluene inhibits the proceeding of the transalkylation reaction between xylene and benzene (generated by deethylation of ethylbenzene), which is the side reaction causing xylene loss. Further, by adding ET to the feedstocks in an amount of 1%, the yield was increased by 0.2% (comparison between Comparative Example 1-B and Example 1-C). This improvement in the yield brings about a very large economic effect in the industrial production in which a large amount of the feedstocks, that is, several tens tons of the feedstocks is processed per hour. Still further, the reaction product obtained in Example 1 has a low concentration of ethylbenzene and a high concentration of *p*-xylene. Thus, it can be seen that this process is very advantageous in the production of *p*-xylene in the "separation-isomerization" cycle, especially in the process wherein adsorptive separation using a zeolite-based adsorbent is used in the p-xylene-separation step.

From the results of Comparative Example 1-A, when the catalyst (Catalyst A) which did not carry a hydrogenating active metal was used, the deethylation activity of ethylbenzene and ethyltoluene was low.

Example 2

In the same manner as in the production of Catalyst B, 20 g of the dried molded product of pentasil zeolite was immersed in 40 ml of an aqueous chloroplatinic acid solution containing 4 mg of Pt in terms of Pt at room temperature, and the mixture was left to stand for 2 hours. The mixture was stirred at intervals of every 30 minutes, and after draining liquid, the product was dried overnight at 120°C. Before using the product in a catalytic reaction, the catalyst was treated with hydrogen sulfide gas flow at 250°C for 2 hours, and then baked at 540°C for 2 hours in the air to obtain Catalyst C. Analysis of the Pt carried in Catalyst C by ICP spectrometry revealed that the amount of the Pt was 169 ppm by weight in terms of Pt.

In a reaction tube, 7.5 g of Catalyst C was packed and the above-described Material oil D was allowed to react under the same conditions as in Example 1. The results are shown in Table 3.

Example 3

In 40 ml of an aqueous palladium chloride solution containing 40 mg of Pd in terms of Pd, 20 g of the dried molded product of the pentasil zeolite which contained ion-exchanged ammonium and calcium ions, which was prepared in the same manner as in Catalyst B was immersed and the mixture was left to stand for 2 hours. The mixture was stirred at intervals of every 30 minutes, and after draining liquid, the product was dried overnight at 120°C. Before using the product in a catalytic reaction, the catalyst was treated with hydrogen sulfide gas flow at 250°C for 2 hours, and then baked at 540°C for 2 hours in the air to obtain Catalyst D. Analysis of the Pd carried in Catalyst D by ICP spectrometry revealed that the amount of the Pd was 1480 ppm by weight in terms of Pd.

In a reaction tube, 7.5 g of Catalyst D was packed and the above-described Material oil D was allowed to react under the same conditions as in Example 1. The results are shown in Table 3.

Example 4

In 40 ml of an aqueous nickel nitrate solution containing 40 mg ofNi in terms of Ni, 20 g of the dried molded product of the pentasil zeolite which contained ion-exchanged ammonium and calcium ions, which was prepared in the same manner as in Catalyst B was immersed and the mixture was left to stand for 2 hours. The mixture was stirred at intervals of every 30 minutes, and after draining liquid, the product was dried overnight at 120°C. Before using the product in a catalytic reaction, the catalyst was treated with hydrogen sulfide gas flow at 250°C for 2 hours, and then baked at 540°C for 2 hours in the air to obtain Catalyst E. Analysis of the Ni carried in Catalyst E by ICP spectrometry revealed that the amount of the Ni was 1680 ppm by weight in terms of Ni.

In a reaction tube, 7.5 g of Catalyst E was packed and the above-described Material oil D was allowed to react under the same conditions as in Example 1. The results are shown in Table 3.

Table 3

| | (Unit: % by weight) | | |
|---|---|---|---|
| | Example 2 | Example 3 | Example 4 |
| Catalyst used | Catalyst C | Catalyst D | Catalyst E |
| Feed material used | Material D | Material D | Material D |
| Degree of Conversion of EB | 90.0 | 90.0 | 88.0 |
| PX/(PX+MX+OX) | 23.5 | 23.5 | 23.5 |
| Et concentration in reaction product | 1.0 | 1.0 | 1.1 |
| Toluene (TOL) concentration in reaction product | 4.4 | 4.5 | 4.0 |

(continued)

| | (Unit: % by weight) | | |
|---|---|---|---|
| | Example 2 | Example 3 | Example 4 |
| Degree of Conversion of ET | 80.2 | 80.0 | 78.5 |
| (PX+MX+OX) Yield | 96.0 | 96.6 | 88.8 |

Example 5

In 40 ml of an aqueous rhenium oxide solution containing 200 mg of Re in terms of Re, 20 g of the dried molded product of the pentasil zeolite which contained ion-exchanged ammonium and calcium ions, which was prepared in the same manner as in Catalyst B was immersed and the mixture was left to stand for 2 hours. The mixture was stirred at intervals of every 30 minutes, and after draining liquid, the product was dried overnight at 120°C. Before using the product in a catalytic reaction, the catalyst was treated with hydrogen sulfide gas flow at 250°C for 2 hours, and then baked at 540°C for 2 hours in the air to obtain Catalyst F. Analysis of the Re carried in Catalyst F by ICP spectrometry revealed that the amount of the Re was 4800 ppm by weight in terms of Re.

In a reaction tube, 7.5 g of Catalyst B or Catalyst F was packed and the above-described Material oil E was allowed to react under the conditions below. The results are shown in Table 4.

Reaction Conditions:

WHSV(hr$^{-1}$): 5.3
Reaction Temperature (°C): 390
Reaction Pressure (MPa): 0.9
$H_2$/Feed(mol/mol): 2.5

Table 4

| (Unit: % bv weight) | | |
|---|---|---|
| Catalyst used | Catalyst B | Catalyst F |
| Feed material used | Material E | Material E |
| Degree of Conversion of EB | 77.9 | 78.1 |
| PX/(PX+MX+OX) | 23.6 | 23.6 |
| Et concentration in reaction product | 1.0 | 0.8 |
| Toluene (TOL) concentration in reaction product | 4.3 | 4.5 |
| Degree of Conversion of ET | 75.1 | 81.0 |
| (PX+MX+OX) Yield | 96.4 | 97.2 |

From Table 4, it can be seen that the larger the amount of the rhenium carried, the higher the recovery of xylene. Material oil E is a simulation of the feedstocks used in the direct feed process omitting the elimination of C9+. It can be seen that the catalyst is effective also in the direct feed process.

Example 6

The concentrations of the non-aromatics in the reaction solution were measured for the reaction solutions obtained by using Catalyst B and Catalyst D in Example 3, respectively, by the method by which the non-aromatics may be analyzed in detail (the analysis conditions described in (2) in Example 1). As a result, as shown in Table 5 below, the concentrations of cyclohexane and methylcyclopentane which were contaminated impurities having the boiling points close to that of benzene were lower in Example 1 in which the Catalyst B was used, and the estimated purity of the benzene final product (benzene purity) calculated by the equation for estimating benzene purity described below was as high as not less than 99.8% by weight in Example 1.

Table 5

| (Unit: % by weight) | | |
| --- | --- | --- |
| Example in which reaction solution was obtained | Example 1-E | Example 3 |
| Catalyst used | Catalyst B | Catalyst D |
| Feed material used | Material D | Material D |
| Benzene concentration in reaction solution | 8.7 | 8.5 |
| n-C6 paraffin concentration in reaction solution | 0.001 | 0.002 |
| Cyclohexane concentration in reaction solution | 0.005 | 0.008 |
| Methylcyclopentane concentration in reaction solution | 0.008 | 0.019 |
| C7 naphthene paraffin concentration in reaction solution | 0.005 | 0.011 |
| Calculated benzene purity* | 99.8 | 99.6 |

Estimated Purity of Final Product Benzene = ([benzene concentration]/a+b+c+d+[benzene concentration]) * 100(%))

wherein a to d represent the following:
a = 0.1 * [n-C6 paraffin concentration]
b = 0.7 * [methylcyclopentane concentration]
c = 1.0 * [cyclohexane concentration]
d = 1.0 * [C7 naphthene paraffin concentration]

**Claims**

1. A process for converting ethylbenzene, comprising the step of bringing a C8 aromatic hydrocarbon mixed feedstocks containing ethylbenzene into contact with an acid type catalyst(s) containing at least one metal selected from the group consisting of the metals belonging to Group VII and Group VIII in the presence of $H_2$ to convert said ethyl-benzene to benzene, said feedstocks containing C9-C10 aromatic hydrocarbons including ethyltoluene, said ethyltoluene being converted to toluene together with said conversion of ethylbenzene.

2. The process according to claim 1, wherein said ethyltoluene in said feedstocks has a concentration of not less than 1% by weight.

3. The process according to claim 2, wherein said ethyltoluene in said feedstocks has a concentration of not less than 3% by weight.

4. The process according to claim 3, wherein said ethyltoluene in said feedstocks has a concentration of not less than 5% by weight.

5. The process according to any one of claims 1 to 4, wherein said ethyltoluene in said feedstocks is converted at a degree of conversion of not less than 50%.

6. The process according to any one of claims 1 to 5, wherein said acid type catalyst contains at least one metal selected from the group consisting of platinum, palladium and rhenium.

7. The process according to claim 6, wherein said acid type catalyst contains rhenium.

8. The process according to claim 7, wherein said rhenium is contained in said acid type catalyst at a content of 0.01% by weight to 5% by weight.

9. The process according to claim 8, wherein said rhenium is contained in said acid type catalyst at a content of 0. 1% by weight to 2% by weight.

10. The process according to any one of claims 1 to 9, further comprising separating, by distillation, the benzene produced by the reaction, and recovering the benzene at a purity of not less than 99.8% by weight.

11. The process according to any one of claims 1 to 10, wherein said acid type catalyst is a pentasil zeolite having a silica/alumina molar ratio of 10 to 70.

12. A process for producing *p*-xylene, comprising the steps of:

   subjecting a C8 aromatic hydrocarbon mixed feedstocks containing ethylbenzene and xylene to said process according to any one of claims 1 to 11 to isomerize said xylene together with converting said ethylbenzene to benzene; and
   separatingp-xylene from the obtained reaction product.

13. A process for producing *p*-xylene, comprising:

   a first deethylation/xylene-isomerization step of subjecting a C8 aromatic hydrocarbon mixed feedstocks containing ethylbenzene and xylene to said process according to claim 12 to isomerize said xylene together with converting said ethylbenzene to benzene;
   a step of separating *p*-xylene from the reaction product obtained in said first deethylation/xylene-isomerization step;
   a step of subjecting xylene contained in the residue after separation to a second xylene-isomerization step to isomerize said xylene; and
   separating *p*-xylene again from the reaction product of said second xylene-isomerization step.

14. The process according to claim 13, wherein said second xylene-isomerization step comprises bringing said residue after separation into contact with an acid type catalyst(s) containing at least one metal selected from the group consisting of the metals belonging to Group VII and Group VIII in the presence of $H_2$.

15. A process for producing *p*-xylene, comprising:

   a deethylation/xylene-isomerization step of subjecting a C8 aromatic hydrocarbon mixed feedstocks containing ethylbenzene and xylene to said process according to claim 12 to isomerize said xylene together with converting said ethylbenzene to benzene;
   a step of separating *p*-xylene from the reaction product obtained in said deethylation/xylene-isomerization step; and
   a step of recycling the residue after separation obtained in said separation step by supplying said residue to said deethylation/xylene-isomerization step.

16. The process according to claim 15, further comprising the step of recycling said residue after separation by mixing said residue with said C8 aromatic hydrocarbon mixed feedstocks and subjecting the resulting mixture to said deethylation/xylene-isomerization step.

17. A process for producing *p*-xylene by using a *p*-xylene-producing equipment for carrying out said first deethylation/ xylene-isomerization step, p-xylene-separation step and second xylene-isomerization step recited in claim 13 or 14, wherein said equipment comprises a bypass line which does not pass through said first deethylation/xylene-isomerization step, and wherein said C8 aromatic hydrocarbon mixed feedstocks mixed with said C9-C10 aromatic hydrocarbons containing ethyltoluene is supplied to said *p*-xylene-separation step, as required.

Fig. 1

Fig. 2

Fig. 3

EP 2 008 988 A1

Fig. 4

## INTERNATIONAL SEARCH REPORT

| International application No. |
|---|
| PCT/JP2007/056547 |

A. CLASSIFICATION OF SUBJECT MATTER
*C07C4/18*(2006.01)i, *B01J29/48*(2006.01)i, *C07C5/27*(2006.01)i, *C07C15/04* (2006.01)i, *C07C15/06*(2006.01)i, *C07C15/08*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C4/18, B01J29/48, C07C5/27, C07C15/04, C07C15/06, C07C15/08, C07B61/00

Documentation searched other than minimum documentation to the extent that such documents are included in the fields searched
Jitsuyo Shinan Koho          1922-1996     Jitsuyo Shinan Toroku Koho     1996-2007
Kokai Jitsuyo Shinan Koho     1971-2007     Toroku Jitsuyo Shinan Koho     1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 04-504577 A  (MOBIL OIL CORP.),<br>13 August, 1992 (13.08.92),<br>Examples 15 to 18<br>& WO 91/08998 A1          & US 4973784 A<br>& WO 91/09824 A1          & AU 9054431 A<br>& AU 9055483 A            & WO 91/11500 A1<br>& AU 9055331 A            & AU 9063488 A<br>& EP 457853 A1            & BR 9007077 A<br>& EP 511207 A1            & EP 512992 A1<br>& JP 5-502012 A           & JP 5-503310 A<br>& WO 92/04306 A1          & AU 9184038 A<br>& EP 546030 A1            & JP 6-500544 A | 1-12<br>13-17 |

|☒| Further documents are listed in the continuation of Box C. | |☐| See patent family annex. |
|---|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>13 June, 2007 (13.06.07) | Date of mailing of the international search report<br>26 June, 2007 (26.06.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2007/056547

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2002-371018 A (Fina Technology, Inc.),<br>26 December, 2002 (26.12.02),<br>Table 1<br>& EP 1260493 A1　　　& US 6504076 B1<br>& CN 1386728 A　　　& US 2003/092950 A1 | 1-6,10,12<br>7-9,11,13-17 |
| X<br><br>Y | JP 10-513498 A (MOBIL OIL CORP.),<br>22 December, 1998 (22.12.98),<br>Page 10, lines 7 to 14; examples<br>& WO 96/24568 A1　　　& IN 189340 A<br>& CA 2209658 A　　　& AU 9650222 A<br>& EP 808299 A1　　　& CN 1179771 A<br>& BR 9607590 A　　　& ZA 9601124 A<br>& US 5763720 A | 1,6,10,12,<br>15,16<br>2-5,7-9,11,<br>13,14,17 |
| X<br>Y | JP 2-91031 A (Teijin Petrochemical Industries,<br>Ltd.),<br>30 March, 1990 (30.03.90),<br>Table 1<br>& EP 361424 A2　　　& US 5032561 A | 1,6,10-17<br>2-5,7-9 |
| X<br>Y | JP 58-77828 A (Toray Industries, Inc.),<br>11 May, 1983 (11.05.83),<br>Examples<br>(Family: none) | 1,6-10,12-17<br>2-5,11 |
| X<br>Y | US 4341622 A (MOBIL OIL CORP.),<br>27 July, 1982 (27.07.82),<br>Column 6, lines 40 to 45; examples<br>(Family: none) | 1,6,10,12<br>2-5,7-9,11,<br>13-17 |
| A | JP 8-119882 A (Chevron USA Inc.),<br>14 May, 1996 (14.05.96),<br>& EP 704416 A1　　　& CA 2132947 A | 1-17 |
| Y | JP 8-143483 A (Mitsubishi Oil Co., Ltd.),<br>04 June, 1996 (04.06.96),<br>(Family: none) | 13,14,17 |
| Y | JP 62-228031 A (Toray Industries, Inc.),<br>06 October, 1987 (06.10.87),<br>& JP 57-200318 A | 13,14,17 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1056626 A
- US 6342649 B
- US 5977429 B
- JP 8143483 A
- JP 60035284 B
- JP 46010064 B
- JP 2002504946 A